(19) Europäisches Patentamt
European Patent Office
Office européen des brevets

(11) **EP 1 891 896 B1**

(12) **EUROPEAN PATENT SPECIFICATION**

(45) Date of publication and mention
of the grant of the patent:
**21.09.2011 Bulletin 2011/38**

(51) Int Cl.:
***A61B 5/07*** *(2006.01)*      ***A61B 5/103*** *(2006.01)*
***A61B 5/00*** *(2006.01)*

(21) Application number: **06119408.0**

(22) Date of filing: **23.08.2006**

(54) **Method and installation for the non-invasive read-out of data of a passive strain sensor with inherent calibration structure**

Verfahren und Anordnung für das nicht-invasive Auslesen von Daten eines passiven Dehnungssensors mit inhärenter Kalibrierstruktur

Méthode et installation pour une lecture non invasive des données d'un capteur de déformation passif avec une structure d'étalonnage inhérente

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**27.02.2008 Bulletin 2008/09**

(73) Proprietor: **Eidgenössische Materialprüfungs- und Forschungsanstalt EMPA 8600 Dübendorf (CH)**

(72) Inventors:
• **Gattiker, Felix 8037 Zürich (CH)**
• **Neuenschwander, Jürg 8624 Grüt (CH)**

• **Sennhauser, Urs Jakob 8305 Dietlikon (CH)**

(74) Representative: **Felber, Josef Felber & Partner AG Dufourstrasse 116 8034 Zürich (CH)**

(56) References cited:
**EP-A1- 1 356 765**      **EP-A2- 0 544 064**
**WO-A2-2005/102151**   **US-A- 3 853 117**
**US-A- 6 034 296**       **US-A1- 2003 100 822**
**US-A1- 2003 176 789**

• **UMBRECHT F ET AL: "A Wireless Implantable Passive Strain Sensor System" SENSORS, 2005 IEEE OCT. 31, 2005, PISCATAWAY, NJ, USA, IEEE, 31 October 2005 (2005-10-31), pages 20-23, XP010899584 ISBN: 0-7803-9056-3**

**Description**

A design of a novel passive strain-sensor for the in-situ measurement of small strains on implants, bones or fixation systems has been presented in WO 2005/102151 A2. The sensing principle is based on hydro-mechanical strain amplification which allows for the abandonment of any electrical circuits. Thus, the sensor can be fabricated applying solely biocompatible or bioresorbable polymeric materials. Finite element simulations are employed to validate the basic sensing principle and to optimize design parameters according to the required target specifications.

A detailed understanding of complex biomechanical motion sequences, forces and moments in the human body is required for the development of improved implants as well as post-surgical therapies, which need to be adapted to the actual healing stage, e.g. bone fracture treatment or osteosynthesis. While wired sensors are acceptable for surgical operations, wireless sensors are preferred for monitoring parameters within the body over longer time periods, in order to prevent skin penetration by wires and minimize the risk of infection. Beside the aim to further miniaturize these sensors and make them bioresorbable in order to avoid surgical removal and promote minimal invasive surgery, an important factor to implement such sensors is to find a way for wireless parameter read-out. Beside applications of such wireless implantable passive strain sensor systems as load/strain monitors for fixations systems or implants, direct in-situ determination of strains on human bone is another important application, e.g. during the time period when patients are executing their rehabilitation training.

The subject of this invention is to provide a method and installation for the remote wireless and passive read-out of the signals of such sensors using advanced ultrasound imaging technologies.

This objective is achieved by a method according to the claim 1.

The objective is further achieved by an installation according to the claim 3.

A possible measurement arrangement is shown in the accompanying figures and the method and installation are being described in relation to these figures. They show:

Figure 1:      The positioning of the sensor on a fixation plate on a fractured bone within the covering tissue and the read-out installation;

Figure 2:      The sensor with its partially filled channels in a top view;

Figure 3:      The sensor with its partially filled channels in a section view seen from its side;

Figure 4:      Illustration of possible ultrasonic echoes obtained;

Figure 5:      A sensor with first calibration structure in a side view and top view;

Figure 6a,b:      The sensor with another calibration structures in a side view and top view.

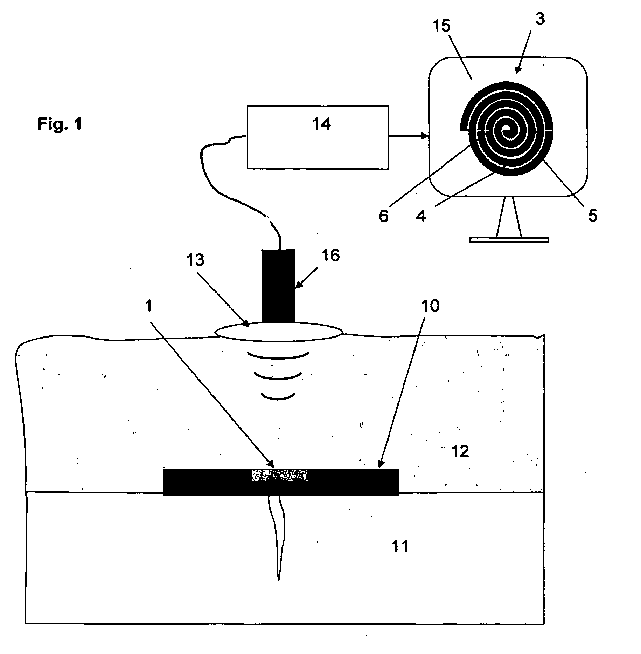

In a specific application, the strain sensor 1 unit will be directly attached to a fixation system 10 which stabilizes the fractured bone 11 during the healing-process, as shown schematically in figure 1. Thus, the strain sensor 1 is embedded into the tissue 12 that covers the bone 11. By using it in a broad range of applications, the sensor 1 could be located anywhere between a couple of millimeters to approximately 10 centimeters beneath the skin. Any load acting on the bone 11, and hence also on the fixation system 10, results in a relative strain typically within the range of $\varepsilon = \Delta l/l_0 < 10^{-2}$ dependent on the moduli of the bone 11 and the fixation system 10 respectively. The sensor signal is read-out remotely in real-time using advanced ultrasound technologies. An ultrasound transducer 16 transmits ultrasonic waves via a coupling medium 13 to the strain sensor 1 which reflects them based on its filling status. For this purpose, the strain sensor 1 contains a reservoir filled with an incompressible liquid. The reservoir is connected to a micro channel system in which the liquid/gas interface moves according to the change of the volume of the reservoir. A calibration between the filling status of the micro channel system and the deformation of the reservoir is obtainable and can be described with a linear function. A small volume change of the reservoir is converted into a large local displacement of the liquid/gas interface within the micro channel system and therefore alters the ultrasonic reflection characteristics of the sensor 1. There is a definite relation between the filling status of the micro channels and the reflection of ultrasonic waves transmitted to these micro channels. The signals are then processed by a microprocessor 14 which leads to an interpretation of the filling status of the spiral-shaped micro channel of the sensor 1 as shown on screen 15. In the displayed representation of the micro channel 3 the same is partly filled with an incompressible liquid 6. The liquid/gas interface 4 indicates to which extent the displayed micro channel 3 is filled. The outer part of the spiral-shaped micro channel 3 beyond the liquid/gas interface 4 is filled with gas 5.

The target strain resolution for the wireless implantable passive strain sensor was defined as $\varepsilon = 10^{-5}$, which corresponds to approximately one percent of maximal strains occurring typically in the stiffest human bones during sportive activities. This resolution can only be achieved if the sensor unit itself _and_ the wireless, non-destructive read-out system provide sufficient sensitivity. In fact the sensor 1 will be covered by an inhomogeneous layer of human tissue 12 of thicknesses up to several centimetres which presents an additional challenge. The advanced ultrasound imaging technology does present a suitable read-out system, since the reflection of the ultrasound waves is highest at material interfaces with high differences in their acoustic impedances as for liquid/gas interfaces 4. The read-out system must be capable of maintaining high accuracy, independent of the sensor's location.

More detailed, the sensor unit consists of two parts, which are the liquid containing reservoir and the micro channel attached to it. The design of the liquid containing reservoir has to be optimized for maximum strain resolution, i.e. maximum change of volume at applied strain. The limited lateral resolution of the ultrasound read-out requires a minimal width of the channel. Under ideal conditions, i.e. the ultrasound wave front is not distorted, the lateral resolution of about 0.5 millimeters required to read-out the wireless implanted passive strain sensor can be easily achieved with a high frequency 3D ultrasound scanner: A 3D image of the sensor's micro channel is acquired with the scanner and an image processing algorithm detects the fluid/gas interface 4 in the micro channel 3. The position of the fluid/gas interface 4 is then related to the strain, which interacts with the sensor.

In a real application a more difficult situation is encountered: By propagating through highly inhomogeneous and damping tissue, the ultrasound beam is considerably distorted and damped resulting in a degraded lateral and axial resolution. The lateral and axial resolution increases with increasing frequencies, but the damping is stronger at higher frequencies. The lower the frequencies are, the better the penetration into the tissue, but the poorer the resolution obtained. A good compromise is found at around 10 MHz. Additionally, high damping of the ultrasound signals results in a poor signal to noise ratio. Even though there exist a lot of sophisticated methods in order to improve the quality of ultrasound signals, there is still no ultrasound technique available which meets the requirements mentioned above. As a result, it is not realistic to achieve the required lateral resolution in order to detect the fluid/gas interface 4 reliably. It is the aim to determine the filling status of the sensor despite a poor resolution. This can be achieved by using the describable relation between the filling status of the micro channel 3 of the sensor 1 and the total reflected ultrasound energy.

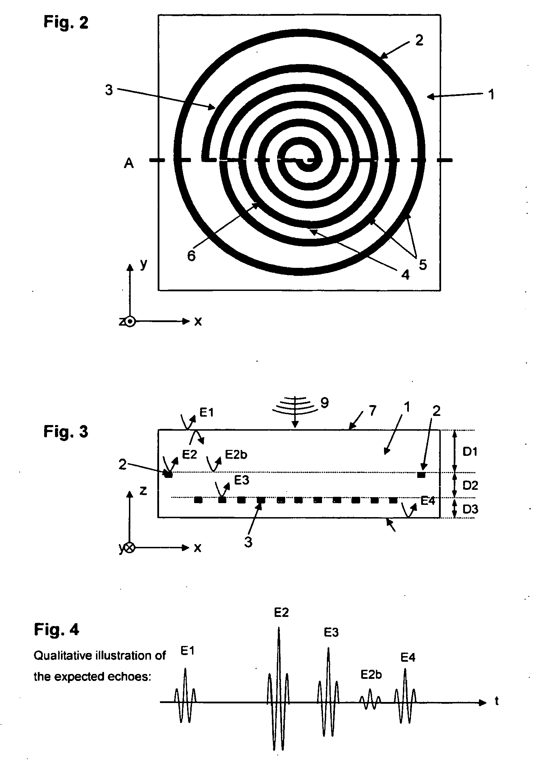

First experiments were conducted with sensors made of Plexiglas. The advantages of evaluating the reflected energy are: This method does not involve the acquisition of a high resolution (lateral) 3D dataset. Thus, the ultrasound pulse frequency can be lowered, which in turn results in a higher penetration depth and it allows faster scanning techniques. But the method requires proper calibration of the received ultrasound signals. This calibration of the ultrasound signals can be achieved by extending the strain sensor with a calibration structure 2 such as shown in figure 2. Since the calibration structure 2 is enclosed by a material 1 with well known constant acoustic properties, its reflected echoes can be used to evaluate the echoes reflected by the micro channel 3. As a result, the ultrasound signals reflected by the strain sensor contain information about the fill level as well as the calibration echoes at the same time. Thus, separate calibration is not necessary anymore. Since all the echo signals contain inherent calibration information, measuring of the fill level becomes independent of the tissue covering the sensor.

More precisely, this new read-out method evaluates the ultrasound energy reflected by the fluid 6/sensor 1 interface and gas 5/sensor 1 interface respectively. This is achieved by the use of a sensor 1 as shown in figure 2 in a top view, having a fillable micro channel 3 and a calibration structure 2. At the center, the micro channel 3 comes out of a reservoir and then forms a spiral. In the actual representation shown, about half of this spiral is filled with the liquid 6. The more the reservoir is squeezed, the more the interface 4 will move toward the outer end of the spiral. A special circle channel 2 filled with a known medium surrounds the spiral channel 3.

In figure 3, the sensor with its fillable micro channel is shown in a section view from its side and in the following, its functioning is explained, whereby the symbols used have this meaning:

$t_P$ =    pulse length in 1
$v_P$ =    sound velocity in 1
$R_1$ =    reflection coefficient at the interface between the sensor's housing (1) and the calibration structure (2)
$L$ =    fill level of the micro channel (3)

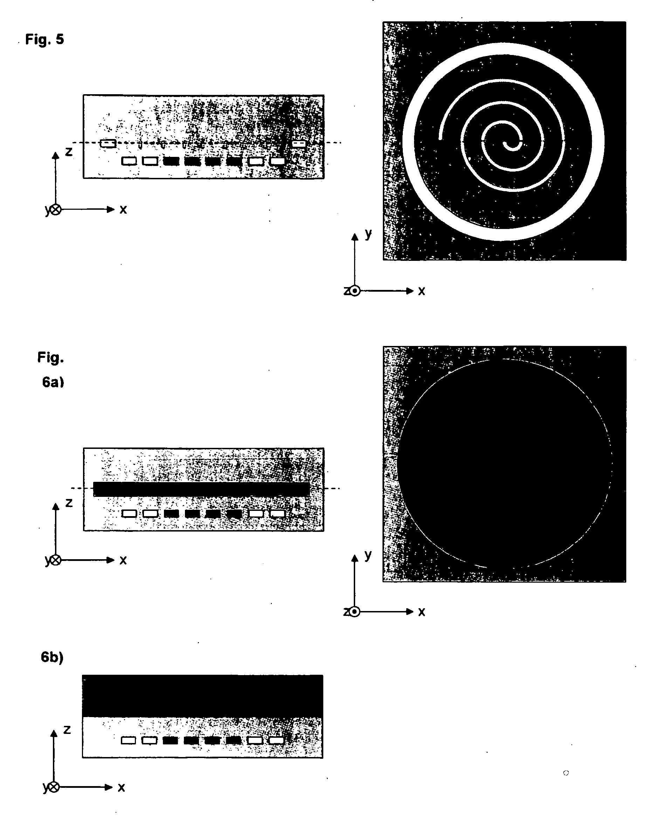

Important characteristics of the sensor are: The calibration structure 2 as well as the micro channel 3 lie on two parallel planes. Looking in negative z-direction, the calibration structure 2 lies above the micro channel 3. The reflection coefficient $|R_1|$ at the interface between the sensor's housing 1 and the calibration structure 2 must be > 0 and $\leq$ 1. These requirements allow several differently shaped calibration structures as later shown in figures 5, 6a and 6b.

The transmitted ultrasonic waves or the insonification angle 9 are approximately perpendicular ($\pm$ 5°) to the front plane 7. The front plane 7 then reflects the first echo E1 followed by the echoes E2, E3 and E4 reflected by the calibration structure 2, the micro channel 3 and the back plane 8 of the sensor 1. Inappropriate sensor dimensions could lead to unwanted interferences between these echoes. Therefore several design rules must be followed in order to prevent relevant echoes to interfere with other ones:

The calibration structure 2 is located in such a way, that its first echo E2 does not interfere with echo E1 reflected by the front plane 7, i.e. D1 ≥ 0.5 $v_P$ $t_P$.

The micro channel 3 is located in such a way, that its first echo E3 does not interfere with echo E2 reflected by the calibration structure 2, i.e. D2 ≥ 0.5 $v_P$ $t_P$. The micro channel 3 is located in such a way, that its first echo E3 does not interfere with echo E4 reflected by the back plane 8, i.e. D3 ≥ 0.5 $v_P$ $t_P$.

The calibration structure 2 is located in such a way, that its multiple echo E2b does not interfere with the first echo E3 of the micro channel 3, i.e. D1 ≥ 2·D2.

Figure 4 shows an illustration of such possible ultrasonic echoes obtained in the time sequence of their detection. It is important to design the sensor in such way as the time sequence between the different types of echoes is large enough in order to clearly separate them and avoid any interferences.

Figure 5 shows another design of a suitable sensor, on the left in a section view, on the right in a top view. Between the front plane 7 and the micro channel 3 a calibration structure 2, e. g. a ring-shaped cavity filled with a gas, is introduced. The calibration structure 2 can have an arbitrary shape as long as it does not overlap the micro channel 3 - overlapping the micro channel would cause unwanted acoustic shadowing, assuming that the insonification 9 direction is perpendicular ± 5° to the front plane 7. These calibration structures are intended to be used when |R₁| = 1.

Figure 6a shows yet another suitable sensor design, on the left in a section view, on the right in a top view. The calibration structure 2 covers the entire micro channel 3. This structure is intended to be used when 0 < |R₁| < 1. Care must be taken when using a calibration structure as shown in figure 6a. Its thickness must be chosen in such a way that the echoes reflected by its front and back plane do not interfere with each other, and multiple echoes reflected by its front and back plane do not interfere with the echoes reflected by the micro channel.

For the read-out of such sensors, the amount of fluid 6/ gas 5 enclosed by the micro channel 3 is being determined by means of ultrasound technology. The insonification 9 direction is always perpendicular ± 5° to the front plane 7. In order to locate the sensor, a low resolution (lateral) 3D image is acquired, e.g. by using a conventional phased array scanner. By evaluating the 3D data, the ultrasonic energy reflected by the calibration structure 2 and the micro channel 3 can be computed. The way to read-out the sensor makes use of the fact that there is a describable relation between the micro channel's 3 reflected acoustic energy and its fill level. The acoustic energy, which is reflected by the calibration structure 2 corresponds to the acoustic energy reflected by the micro channel 3 having a known (constant) fill level, independent of the tissue covering the sensor. Therefore, by measuring the acoustic energy reflected by the micro channel 3, its fill level can be computed.

In a specific approach, the method for the non-invasive read-out of data of a passive strain sensor follows these specific steps:

a) moving a focused ultrasound beam generated by an ultrasound transducer (e.g. a pulsed piezo crystal, capacitive micromachined ultrasound transducers (CMUT)) along predetermined paths in order to scan the entire sensor structure;

b) acquiring the reflected ultrasound echo signals of all these paths;

c) using the acquired data set, the echoes reflected by the micro channel(s) and those reflected by the calibration structure are extracted and the total reflected sonic energy ($A_M$) of the micro channel(s) (3) and the total reflected sonic energy ($A_C$) of the calibration structure (2) is determined;

d) calibrating the obtained signals in order to compensate the influence of the material between the ultrasound probe and the passive strain sensor, by comparing the reflected echoes of the micro channel(s) with those of the calibration structure, by computing the quotient (Q) of the micro channel's total reflected energy ($A_M$) and the calibration structure's total reflected energy ($A_C$)

$$Q = \frac{A_M}{A_C};$$

e) determining the strain-depended fill level of the micro channel, based on the ratio (Q) between the reflected energy of the calibration structure ($A_C$) and the reflected energy of the micro channel ($A_M$), whereby the ratio Q is conceived to be a function $f$ of the micro channel's fill level, which can be approximated with a straight line:

$$\frac{A_M}{A_C} = f(Fill\ Level) \approx a \cdot Fill\ Level + b.$$

Thus:

$$Fill\ Level \approx \left( \frac{A_M}{A_C} - b \right) \cdot \frac{1}{a}$$

whereas the fill level is defined to be the length of the filled part divided by the total length of the micro channel, so as the fill level is always in the range between 0 and 1.

It is the ratio $\dfrac{A_M}{A_C}$ which ultimately indicates the filling status of micro channel of the sensor 1. *a* and *b* are found by evaluating the straight line and therefore providing a sufficient calibration. By the calibration, the signals eventually become independent on the depth location of the sensor within the tissue.

## Claims

1.  Method for the non-invasive read-out of data of a passive strain sensor (1) which is covered by sound permeable material (12) and whereby the sensor (1) consists of a flat housing incorporating one or more micro channels (3) filled with a strain-dependent quantity of fluid (6) or gas (5) and having a calibration structure (2), comprising the following steps:

    a) moving a focused ultrasound beam along predetermined paths in order to scan the entire sensor structure (1);
    b) acquiring the reflected ultrasound echo signals of all these paths;
    c) using the acquired data set, whereby the echoes reflected by the micro channel(s) (3) and those reflected by the calibration structure (2) are extracted;
    d) processing the obtained signals in order to compensate the influence of the material (12) between the ultrasound probe and the passive strain sensor (1), by using the signals from the calibration structure (2) to calibrate the echoes of the micro channel(s) (3);
    e) determining the strain-depended fill level of the micro channel (3), based on the linear relation between the fill level of the micro channel (3) and the reflected and calibrated sonic energy.

2.  Method for the non-invasive read-out of data of a passive strain sensor according to claim 1,

    a) moving a focused ultrasonic beam generated by an ultrasound transducer (16) (e.g. a pulsed piezo crystal, capacitive micromachined ultrasound transducers (CMUT)) along predetermined paths in order to scan the entire sensor structure (1);
    b) acquiring the reflected ultrasound echo signals of all these paths;
    c) computing the integrated echo amplitudes ($A_M$) and ($A_C$) of the micro channel(s) (3) and the calibration structure (2) respectively;
    d) calibrating the obtained signals in order to make them independent of the material (12) between the ultrasound transducer (16) and the passive strain sensor (1), by computing the quotient (Q) of the micro channel's (3) integrated echo amplitudes ($A_M$) and the calibration structure's (2) integrated echo amplitudes ($A_C$)

$$Q = \frac{A_M}{A_C}\ ;$$

    e) determining the strain-depended fill level of the micro channel, based on the ratio (Q) between the integrated echo amplitudes of calibration structure ($A_C$) and the integrated echo amplitudes of the micro channel ($A_M$), whereby the ratio Q is conceived to be a function *f* of the micro channel's fill level, which can be approximated with a straight line:

$$\frac{A_M}{A_C} = f(\text{Fill Level}) \approx a \cdot \text{Fill Level} + b$$

Thus:

$$\text{Fill Level} \approx \left(\frac{A_M}{A_C} - b\right) \cdot \frac{1}{a}$$

whereas the fill level is defined to be the length of the filled part divided by the total length of the micro channel, so as the fill level is always in the range between 0 and 1.

3. An installation for the non-invasive read-out of data of a passive strain sensor (1) built into a sound permeable material (12), comprising a sensor (1) consisting of a flat housing incorporating one or more micro channels (3) filled with a strain-dependent quantity of fluid (6) or gas (5), and comprising

• An ultrasonic transducer (16) connected to a pulser for generating a focused ultrasonic beam;
• the at least one micro channel (3) filled with a strain-dependent quantity of fluid (6)/gas (5), this micro channel (3) laying on a plane parallel to the sensor's (1) surface, whereas it has an arbitrary shape, e.g. a spiral;

**characterized in that** it further comprises

• a XYZ scanner, by which the focused ultrasonic beam is moveable along a predetermined path over the sensor (1) to be read out;
• further the sensor (1) including at least one calibration structure (2) laying on a parallel plane to the plane of the micro channel (3) so that its reflected ultrasound echoes are clearly separable from the ones reflected by the micro channel(s) (3) and interferences between these echoes are avoided in case of perpendicular insonification; and
• a receiver for the ultrasonic signals reflected by the micro channel(s) and the calibration structure, connected to a microprocessor (14) for further signal processing.

4. An installation according to claim 3, wherein the sensor (1) built into the sound permeable material (12) is flat, and the reflection coefficient between the calibration structure (2) and the flat sensor's (1) housing is approximately 1 or -1, and the micro channel (3) - at a chosen position of the ultrasonic transducer - is located outside of the acoustic shadow of the calibration structure (2) or vice versa in case of perpendicular insonification.

5. An installation according to claim 3, wherein the sensor (1) built into the sound permeable material (12) is flat, and the reflection coefficient between the calibration structure (2) and the flat sensor's (1) housing is $\neq$ -1, $\neq$ 0 and 1, and the micro channel (3) - at a chosen position of the ultrasonic transducer - is located inside the acoustic shadow of the calibration structure (2), and the multiple echoes occurring in the calibration structure (2) are clearly separable from the echoes reflected by the micro channel (3).

**Patentansprüche**

1. Verfahren für das nicht-invasive Auslesen von Daten aus einem passiven Deformations-Sensor (1), der von einem Schall-durchlässigen Material (12) bedeckt ist, wobei der Sensor (1) aus einem flachen Gehäuse besteht, das einen oder mehrere Mikro-Kanäle (3) enthält, die gefüllt sind mit einer dehnungs-abhängigen Menge von Flüssigkeit (6) oder Gas (5), und wobei eine Kalibrierstruktur (2) vorhanden ist, wobei das Verfahren die folgenden Schritte einschliesst:

a) Bewegen eines fokussierten Ultraschall-Strahls längs von vorbestimmten Wegen, um die ganze Sensorstruktur (1) abzuscannen;
b) Aufnahme der reflektierten Ultraschall Echosignale über all diese Wege;
c) Verwenden des aufgenommenen Daten-Sets, wobei die vom Mikro-Kanal/bzw. von den Mikro-Kanälen (3) sowie die von der Kalibrierstruktur (2) reflektierten Echos extrahiert werden;

d) Verarbeiten der erhaltenen Signale, um den Einfluss des Materials (12) zwischen der Ultraschall-Probe und dem passiven Dehnungssensor (1) zu kompensieren, indem die Signale von der Kalibrierstruktur (2) benützt werden, um die Echos vom Mikro-Kanal/bzw. von den Mikro-Kanälen (3) zu kalibrieren;

e) Bestimmen des dehnungs-abhängigen Füllgrades der Mikrokanäle (3), basierend auf der linearen Beziehung zwischen dem Füllgrad der Mikrokanäle (3) und der reflektierten und kalibrierten Ultraschall-Energie.

2. Verfahren für das nicht-invasive Auslesen von Daten aus einem passiven Deformations-Sensor (1) nach Anspruch 1,

a) Bewegen eines fokussierten Ultraschall-Strahls, der von einem Ultraschall-Umwandler (16), (zum Beispiel einem gepulsten Piezo-Kristall, einem kapazitiven mikromaschinellen Ultraschall Umwandler (CMUT) erzeugt wird), längs von vorbestimmten Wegen, um die ganze Sensor-Struktur (1) abzuscannen;

b) Aufnahme der reflektierten Ultraschall Echosignale über all diese Wege;

c) Berechnen der integrierten Echo-Amplituden (AM) und (Ac) des MikroKanals/der Mikrokanäle (3) bzw. der Kalibrier-Struktur (2);

d) Kalilbrieren der erhaltenen Signale, um sie unabhängig vom Material (12) zwischen dem Ultraschall-Umwandler (16) und dem passiven Dehnungssensor (1) zu machen, indem der Quotient (Q) berechnet wird, aus den integrierten Echo-Ampliduden (AM) der Mikrokanäle (3) und den integrierten Echo-Amplituden ($A_C$) der Kalibrierstruktur (2):

$$Q = \frac{A_M}{A_C} ;$$

e) Bestimmen des dehnungs-abhängigen Füllgrades des Mikrokanals, basierend auf der Relation (Q) zwischen den integrierten Echo-Amplituden ($A_C$) der Kalibrierstruktur und den integrierten Echo-Amplituden (AM) des Mikrokanals, wobei die Relation Q als Funktion $f$ des Füllgrades des Mikrokanals betrachtet wird, welcher approximativ durch eine gerade Linie repräsentiert wird, nach folgender Beziehung:

$$\frac{A_M}{A_C} = f(F\ddot{u}llGrad) \approx a \cdot F\ddot{u}llGrad + b$$

und daher:

$$F\ddot{u}llGrad \approx \left( \frac{A_M}{A_C} - b \right) \cdot \frac{1}{a}$$

wobei der Füllgrad definiert wird durch die Länge des gefüllten Teils geteilt durch die totale Länge des Mirkokanals, sodass der Füllgrad stets eine Zahl zwischen 0 und 1 ist.

3. Vorrichtung für das nicht-invasive Auslesen von Daten aus einem Dehnungssensor (1), eingebaut in ein Schall-durchlässiges Material (12), wobei die Vorrichtung einen Sensor (1) einschliesst, mit einem flachen Gehäuse, das einen oder mehrere Mikrokanäle (3) enthält, die gefüllt sind mit einer dehnungs-abhängigen Menge von Flüssigkeit (6) oder Gas (5), und wobei die Vorrichtung weiter einschliesst:

• einen Ultraschall-Umwandler (16), der an einen Pulsgenerator angeschlossen ist, zum Generieren eines fokussierten Ultraschall-Strahls;

• wenigstens einen Mikro-Kanal (3), der gefüllt ist mit einem dehnungsabhängigen Füllgrad einer Flüssigkeit (6) oder eines Gases (5), wobei dieser Mikro-Kanal (3) in einer Ebene liegt, die parallel zur Oberfläche des Sensors (1) verläuft, und wobei er eine beliebige Form aufweist, zum Beispiel eine Spirale; **dadurch gekennzeichnet, dass** die Vorrichtung weiter einschliesst:

• einen XYZ Scanner, durch den der fokussierte Ultraschall-Strahl längs eines vorbestimmten Weges über dem Sensor (1), der ausgelesen werden soll, bewegbar ist;
• weiter einen Sensor (1), der wenigstens eine Kalibrier-Struktur (2) einschliesst, die parallel zur Ebene eines Mikrokanals (3) verläuft, sodass seine reflektierten Ultraschall-Echos klar separierbar sind von jenen, die durch den oder die Mikrokanäle (3) reflektiert werden und auch Interferenzen zwischen diesen Echos verhindert werden, im Fall von rechtwinkliger Beschallung; und
• einen Empfänger für die Ultraschall-Signale, die von dem oder den Mikrokanälen reflektiert werden, und eine Kalibrierstruktur, verbunden mit einem Mikroprozessor (14) für die weitere Verarbeitung der Signale.

**4.** Vorrichtung nach Anspruch 3, wobei der Sensor (1), der in das Schalldurchlässige Material (12) eingebaut ist, flach ist, und wobei der Reflektionskoeffizient zwischen der Kalibrierstruktur (2) und dem Gehäuse des flachen Sensors (1) etwa 1 oder -1 beträgt, und dass der Mikrokanal (3) - bei einer gewählten Position des Ultraschall-Umwandlers - ausserhalb des akustischen Schattens der Kalibrierstruktur (2) liegt, oder umgekehrt im Fall einer rechtwinkligen Beschallung.

**5.** Vorrichtung nach Anspruch 3, wobei der Sensor (1), der in das Schalldurchlässige Material (12) eingebaut ist, flach ist, und wobei der Reflektionskoeffizient zwischen der Kalibrierstruktur (2) und dem Gehäuse des flachen Sensors (1) $\neq$ -1, $\neq$ 0 und $\neq$ 1 ist, und der Mikrokanal (3) - bei einer gewählten Position des Ultraschall-Umwandlers - innerhalb des akustischen Schattens der Kalibrierstruktur (2) liegt, und die mehrfachen Echo, die in der Kalibrierstruktur (2) aufreten, klar separierbar sind von den Echos, die von den Mikrokanälen (3) reflektiert werden.

## Revendications

**1.** Procédé destiné à la lecture non invasive de donnés en provenance d'un capteur (1) passif de déformation , lequel capteur est couvert par un matériau (12), qui laisse passer le son, où le capteur (1) est constitué d'un boîtier plat, qui contient un ou plusieurs micro - canaux (3) , qui est rempli respectivement qui sont remplis d'une quantité de liquide (6) ou de gaz (5), laquelle quantité étant dépendant de la dilatation dudit liquide respectivement dudit gaz, et où existe une structuré de calibration (2) et où ledit procédé comprend lesdites étapes:

a) déplacement d'un rayon ultrason focalisé le long de trajets prédéterminés pour pouvoir scanner toute la structure (1) du capteur;
b) réception des signaux écho d'ultrason réfléchis sur tous ces trajets;
c) utilisation du set de données reçu, où les échos qui sont réfléchis par le micro - canal respectivement par les micro-canaux (3) ainsi que par la structure de calibrage sont extraits;
d) traitement des signaux obtenus pour compenser l'influence du matériau (12) entre l'échantillon d'ultrason et le capteur passif de dilatation (1), en utilisant les signaux en provenance de la structure de calibrage (2), pour calibrer les échos du micro-canal respectivement des micro-canaux (3);
e) détermination du degré de remplissage, qui dépend de la dilatation, des micro - canaux, sur la base de la relation linéaire entre le degré de remplissage des micro-canaux (3) et l'énergie d'ultrason réfléchie et calibrée.

**2.** Procédé destiné à la lecture non invasive de donnés en provenance d'un capteur (1) passif de déformation, lequel capteur est couvert par un matériau (12), selon la revendication 1, où ledit procédé comprend lesdites étapes:

a) déplacement d'un rayon ultrason focalisé, qui est généré par un convertisseur d'ultrasons (comme par exemple par un cristal piézoélectrique pulsé, un transducteur ultra-sonore micro - usiné capacitif le long de trajets prédéterminés pour pouvoir scanner toute la structure (1) du capteur;
b) réception des signaux écho d'ultrason réfléchis sur tous ces trajets;
c) calcul des amplitudes des échos intégrées ($A_M$) et ($A_C$) du micro-canal respectivement des micro-canaux (3) ainsi que de la structure de calibrage (2);
d) calibrage des signaux obtenus, pour les rendre indépendants du matériau entre le convertisseur d'ultrasons (16) et le capteur de dilatation passif (1), en calculant le quotient (Q) à partir des amplitudes des échos intégrées ($A_M$) et ($A_C$) du micro-canal respectivement des micro-canaux (3) ainsi que de la structure de calibrage (2):

$$Q = A_M / A_C$$

e) détermination du degré de remplissage , qui dépend de la dilatation, du micro - canal, sur la base de la relation (Q) entre les amplitudes intégrées des échos (Ac) de la structure de calibrage et les amplitudes intégrées des échos ($A_M$) du micro - canal, où la relation Q est considérée comme une fonction f du degré de remplissage du micro - canal, lequel canal est représenté de façon approximative par une ligne droite, selon la relation suivante

$$A_M / A_C = f \text{ (degré de remplissage )} \approx a * \text{degré de remplissage} + b$$

et donc:

$$\text{degré de remplissage} \approx ( A_M / A_C - b) * 1/a$$

où le degré de remplissage est défini par la longueur de la partie remplie divisée par la longueur totale du micro - canal, de manière à ce que le degré de remplissage soit toujours un chiffre entre 0 et 1.

3. Dispositif destiné à la lecture non invasive de donnés en provenance d'un capteur (1) de déformation , lequel capteur est incorporé dans par un matériau (12) , qui laisse passer le son, où le dispositif comprend un capteur (1), qui est constitué d'un boîtier plat, qui contient un ou plusieurs micro - canaux (3) , qui est rempli respectivement qui sont remplis d'une quantité de liquide (6) ou de gaz (5), et où ledit dispositif comprend en outre:

• un convertisseur d'ultrasons (16), qui est connecté à un générateur d'impulsions, pour générer un rayon d'ultrason focalisé
• au moins un micro - canal (3) , qui est rempli d'un degré de remplissage, qui dépend de la dilatation du liquide respectivement du gaz, d'un liquide (6) ou d'un gaz (5), où ce micro-canal (3) est situé dans un plan qui s'étend de façon parallèle par rapport à la surface du capteur (1) et où ledit micro-canal présente une forme quelconque qui est par exemple une spirale; **caractérisé en ce que** le dispositif comporte en outre:

• un scanner x - y - z, à travers lequel le rayon d'ultrason focalisé peur être déplacé le long d'un trajet prédéterminé via le capteur (1) qui doit être lu;
• en outre un capteur (1), qui comprend au moins une structure de calibrage (2), qui s'étend de façon parallèle par rapport à un plan d'un micro-canal (3), de maniéré à ce que les échos d'ultrason réfléchis dudit micro-canal peuvent être séparés des échos qui sont réfléchis par le micro-canal ou les micro-canaux (3) et **en ce que** des interférences entre ce échos sont évités, dans le cas d'une insonification perpendiculaire; et
• un récepteur pour le signaux d'ultrason qui sont réfléchis par le micro-canal respectivement par les micro-canaux, et une structure de calibrage, qui est connectée à un microprocesseur (14) pour le traitement ultérieur des signaux.

4. Dispositif selon la revendication 3, où le capteur (1), qui est incorporé dans la matériau (12) qui lasse passer le son, est plat, et où le coefficient de réflexion entre la structure de calibrage (2) et le boîtier du capteur (1) plat s'élève à 1 ou -1, et en ce que le micro-canal (3) - lors d'une position choisie du convertisseur d'ultrasons - est situé à l'extérieure de l'ombre acoustique de la structure de calibrage (2) ou vice versa dans le cas d'une insonification perpendiculaire.

5. Dispositif selon la revendication 3, où le capteur (1), qui est incorporé dans la matériau (12) qui laisse passer le son, est plat, et où le coefficient de réflexion entre la structure de calibrage (2) et le boîtier du capteur (1) plat s'élève à $\neq$ -1 , $\neq$ 0 et $\neq$ 1, et en ce que le micro - canal (3) - lors d'une position choisie du convertisseur d'ultrasons - est situé à l'intérieure de l'ombre acoustique de la structure de calibrage (2) et en ce que les échos multiples, qui sont générés dans la structure de calibrage (2) peuvent être séparés de façon claire des échos, qui sont réfléchis par les micro-canaux (3).

**Fig. 1**

**Fig. 2**

**Fig. 3**

**Fig. 4**

Qualitative illustration of
the expected echoes:

Fig. 5

Fig.
6a)

6b)

**EP 1 891 896 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005102151 A2